# EUROPEAN PATENT APPLICATION

(11) **EP 2 594 831 A1**
(43) Date of publication of application: **22.05.2013**
(21) Application number: 12193041.6
(22) Date of filing: 16.11.2012
(51) Int. Cl.: F16K 7/02, F16K 31/06, F16K 1/12

(54) **An expiratory valve for controlling a flow**

(30) Priority: 17.11.2011 US 201161561226 P; 17.11.2011 EP 11189644
(71) Applicant: Mindray Medical Sweden AB, 174 57 Sundbyberg (SE)
(72) Inventor: Cewers, Göran, 216 42 Limhamn (SE); Ringdén, Ola, 172 37 Sundbyberg (SE); Werner, Johan, 142 52 Skogås (SE)
(74) Representative: KIPA AB

(57) **Abstract**

A valve (3) for a medical ventilator comprising a hollow flexible body (39) having at least one outwardly bulging area (31a, 31b) with a curved inner surface. The hollow flexible body (39) further comprises an inwardly protruding abuttable area (32) positioned between two fixed end points (33a, 33b). Further the valve comprises a flow channel located inside said hollow flexible body (39) for passage of a fluid between an inlet and an outlet, a valve seat (30) centrally positioned in said flow channel and an annular element (31) circumferentially arranged around said hollow flexible body (39). The annular element (31) is arranged to be movable in an axial direction along the flow channel by an actuator unit, and whereby said abuttable area (32) is at least partially repositioned and/or reshaped to control a flow of the fluid through the flow channel.

## Description

### BACKGROUND OF THE INVENTION

### Related applications

The present application relates to the following application of the same inventor as the present application with the following title: "VALVE AND METHOD TO CONTROL A FLOW" (PCT/EP2011/057810); which all are incorporated herein by reference in their entirety for all purposes

### Field of the Invention

The invention pertains in general to the field of valves. More particularly, the invention relates to a valve device for mechanically controlling the flow of at least one fluid through at least one channel. Even more particularly, the invention relates in some embodiments to expiratory valves for breathing machines like medical ventilators or mechanical ventilators.

### Description of the Prior Art

It is known that when designing low pressure valves, especially in the field of gas control in medical ventilators, is it of high importance that the flow channel in the valve has low flow resistance and no or little turbulence. Also, in some applications, requirements are high concerning cleaning of contaminated parts of the valve. Moreover, it is often desirable for the design of the valve to be small and light, and that the actuator controlling the valve may be made small and isolated from the flow channel.

Examples of low pressure valve applications are expiration valves, patient pressure relief valves, mixer valves, and flow valves in low pressure systems.

Today, the commonest design of low pressure valves comprises a circular disk lying against the end of a tube forming a valve seat. US patent 5,127,400 discloses an example of such a design. The drawbacks of such a design are the complexity of the flow channel, which causes turbulence and cleaning issues. Moreover, the entire circular disk is exposed to a pressure, while the flow only depends on the outer edge of the disk. Thus an unnecessarily strong, heavy and expensive actuator is needed to control this type of valve.

Another example is international patent application WO 2008/112332 disclosing an inline valve. The inline valve includes a housing, a choke member in operable communication with the housing, a portion of the choke member being substantially immobile relative to the housing, and a portion of the choke member being mobile relative to the housing. The inline valve further includes, an actuator in operable communication with the movable portion of the choke member, the actuator selectively causing the choke member to deform radially. However, the valve disclosed in WO 2008/112332 only uses one area that can be deformed and can therefore not be used as a valve device between two fixed end positions. Further, the valve can only choke a flow from the inside against a hard conduit. WO 2008/112332 does not disclose anything regarding how to avoid deformation and wear nor how to be able to simply clean or disassemble the valve. Thus the disclosed valve is not suitable for medical devices.

United States patent number 5,119,861 discloses a normally closed valve for controlling flow of fluid material through a straight, non-tapered conduit, which has resilient elastomeric seal attached to the perimeters of two axially aligned rigid end plates. Elasticity of elastomeric seal draws end plates together while radially expanding so as to engage and seal against the inner wall of conduit. This valve may be opened by axially separating the two opposing end plates using a locally actuated displacer mechanism attached to one of the end plates, causing the elastomeric seal to radially contract and the valve to open. The issues with the design of this valve are almost the same as for WO 2008112332. Even though US 5,119,861 disclose a valve having at least two resilient members, the configuration of the device makes it only usable when one end is displaceable. Thus it cannot be used in-between two fixed end-points. Since the valve is normally closed, the focus and the use of more than one resilient member are to increase the sealing effect for specific applications.

Hence, an improved valve would be advantageous and, in particular an actuator-controlled valve allowing for increased flexibility, an improved control of the closing and opening, cost-effectiveness, and/or fulfilling the above-mentioned criteria, of a small and light actuator-controlled valve having low flow resistance and no or little turbulence, would be advantageous.

### SUMMARY OF THE INVENTION

Accordingly, embodiments of the present invention preferably seek to mitigate, alleviate or eliminate one or more deficiencies, disadvantages or issues in the art, such as the above-identified, singly or in any combination by providing a device and a method, for controlling a flow through a valve, according to the appended patent claims.

According to one aspect, a valve for a medical ventilator is described. The valve comprises a hollow flexible body having at least one outwards bulging area with a curved inner surface. The hollow flexible body further comprises an inwardly protruding abuttable area positioned between two fixed end points. Further the valve comprises a flow channel located inside said hollow flexible body for passage of a fluid between an inlet and an outlet, a valve seat centrally positioned in said flow channel and an annular element circumferentially arranged around said hollow flexible body. The annular element is arranged to be movable in an axial direction along the flow channel by an actuator unit, and whereby said abuttable area is at least partially repositioned and/or reshaped to control a flow of the fluid through the flow channel. In some examples of the valve, the bulging area of the hollow flexible body is positioned upstream the abuttable area. The may help to increase the movability of the annular element without causing stress or strain in the material of the hollow flexible body. This may further provide an improved opening or closing response of the valve depending on the positing of the abuttable area in relation to the valve seat.

In some examples, the bulging areas of the hollow flexible body have curved inner surfaces. Additionally in some examples, the hollow flexible body has an increased inner diameter at the bulging areas. Additionally and/or alternatively, in some examples, the bulging areas may have a substantially constant wall thickness at a central portion of the bulging area.

Further examples of the valve are defined in the dependent claims, wherein features for the second and subsequent aspects of the invention are as for the first aspect mutatis mutandis.

An advantage of the described valve is to facilitate the positioning of a valve between two non-flexible in and outlet channels to perform opening and closing movements of the valve without causing strain on the material. Further, this avoids strains on the mountings of the non-flexible in- and outlet channels. Strain or stress could cause lasting deformation or wear. For medical use, wear such as cracks in the conduit could be an issue since it may increase the risk for contamination. Deformation of wear may also affect the reliability of the equipment, e.g., the valve may break down in critical situations. Such drawbacks are effectively avoided by the valve mechanism described herein.

A further advantage is that construction materials of the hollow flexible body and the valve seats may be autoclavable and/or the construction material in the hollow flexible body and the valve seats may be disposable. Also, the valve may comprise parts being autoclavable combined with parts being disposable. Examples of such autoclavable materials include silicone rubber, stainless steel etc.

Even further, an advantage of the valve design is the simplicity in dissembling and assembling the different portions which could be crucial in medical applications. Further advantageous is that those parts that may be hard to clean, and/or expensive to manufacture, e.g., the actuator unit, is kept separate from the flow channel with a small possibility of being contaminated.

Another advantage of the valve design is that the flow channels may thus be provided with low flow resistance and low pressure drop due to its fluid-dynamic shape and low flow turbulence. This is particularly important in medical ventilator, such as expiratory valve applications, as expiration normally is performed passive by the elasticity of the patient's chest and work of breathing is to be reduced.

In some examples of the valve the abuttable area, upon axial movement of the annular element, is configured to be in a radially collapsed state when sealingly abutting said valve seat and/or to be in an uncollapsed state when said valve is open.

Further, in some examples, the abuttable area has diamond shaped zones with a central notch.

Moreover, in some examples, the notch of the diamond shaped zones is configured to provide a twining shape along the inner diameter of the abuttable area, when in a collapsed state. In some examples, the twining shape is sinusoidal.

An advantage with the twined shape when collapsed is increased control over the collapse. Also, the collapse may be conducted without compressing the material of the hollow flexible body when choking and/or closing the valve. Further, an advantage of the twinned shape when collapsed may be improved sealable effect when the notch of the abuttable area completely abuts the valve seat.

Also, in some examples of the valve a tubular element may extend into the flow channel downstream and/or upstream. The tubular element may have an end surface arranged to abut an inner wall of the bulging area or to abut the abuttable area when the valve is in an open state.

This arrangement has the advantage that fluid may be prevented from entering the bulging area when the valve is open. Thus, less turbulence in the flow of a fluid through the flow channel of the valve may be obtained.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which
Figs. 1A to 1C are showing an exemplary embodiment according to the principle of the invented valve mechanism;
Figs. 2A and 2B are showing another exemplary embodiment according to the invented valve mechanism;
Figs. 3A and 3B are showing a further exemplary embodiment according to the invented valve mechanism;
Figs. 4A and 4B are showing an exemplary embodiment according to the invented valve mechanism;
Figs. 5A to 5C are showing an exemplary embodiment of the invention according to the invented valve mechanism;
Figs. 6A and 6B are showing a exemplary embodiment of the invented valve mechanism;
Figs. 7A and 7E are showing exemplary embodiments of an area configuration with diamond shapes according to the invented valve mechanism;
Fig. 8 is showing an exemplary embodiment of the invented valve mechanism with an area having diamond shaped zones.

### DESCRIPTION OF THE PREFERRED EMBODIMENTS

Specific examples of the invention will now be described with reference to the accompanying drawings. This invention may, however, be embodied in many different forms and should not be construed as limited to the examples set forth herein; rather, these examples are provided so that this disclosure will be thorough and complete, and will fully convey the scope of the invention to those skilled in the art. The terminology used in the detailed description of the examples illustrated in the accompanying drawings is not intended to be limiting of the invention. In the drawings, like numbers refer to like elements.

The following description focuses on a valve to control a flow of a fluid through a flow channel. In particular the valve may be used in breathing machines or medical ventilators. An example of such a valve is an expiratory valve. However, it will be appreciated that the invention is not limited to this application but may be applied to many other mechanical valves to control a flow.

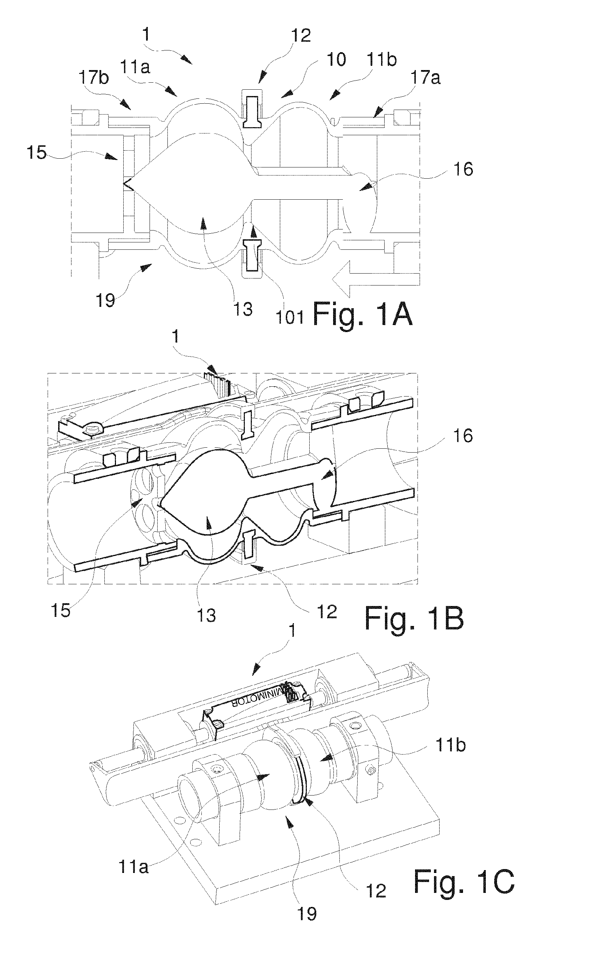

In an example of a valve according to Figs. 1A to 1C, the valve 1 is made of a hollow flexible body 19. Fig. 1A shows a cross-sectional view of the valve 1.

An advantageous design of a valve 1 according to one example is to make the hollow flexible body 19 as a soft conduit made of materials such as silicone.

The flexible body 19 has in this illustrated embodiment two outwards bulging areas 11a and 11b. In one example, the bulging areas aa1 and 11b have curved inner surfaces. Additionally in some examples, the hollow flexible body 19 has an increased inner diameter at the bulging areas 11a and 11b. Additionally and/or alternatively, in some examples, the bulging area 11a and 11b may have a substantially constant wall thickness at a central portion.

Between the two outwardly bulging areas 11a and 11b is a waist area 10 located, whereby a generally dumbbell shaped profile is obtained.

The flexible hollow body 19 of the valve 1 is fastened at two fixed endpoints 17a and 17b.

Inside the hollow flexible body 19 is a flow channel arranged having a centrally positioned valve seat 13. In some examples a valve seat 13 has a rotationally symmetric circular profile. Alternatively, in some embodiments the valve seat 13 has a rotationally symmetric conical profile. This design has the advantage of further decreasing the turbulence of the flow due to the conical shape.

The valve seat 13 is positioned and fixed using at least one strut 15, 16. Alternatively, two struts 15, 16 may be used to fix the valve seat 13 in the flow channel.

Further, an annular element 12 is arranged to circumferential the hollow flexible body 19. In some examples the annular element 12 is circumferentially arranged around at least a portion of the waist area 10. The annular element 12 may be moved in an axial direction of the flow. The axial movement of the annular element 12 may be conducted by using an actuator unit, such as a piezoelectric unit or a minimotor.

When moving the annular element 12 axially either towards or away from the valve seat, the flow of a fluid through the flow channel may be controlled. The flow of a fluid through the flow channel is in the direction of the arrow.

The movement of the annular element 12 causes an abutting area 11 of the hollow flexible body 19 to be repositioned or reshaped. In Figs. 1A to 1C, an axial movement of the annular element 12 in a direction towards the upstream part of valve seat 13 may decrease the flow through the flow channel. An axial movement of the annular element 12 in a direction away from the valve seat 13 will increase the flow through the flow channel. The increase or decrease of the flow is due to the change in the flow cross-section area of the flow channel between the inwardly protruding abuttable area 101 and an upstream area of the valve seat 13. The change is caused by the repositioning and/or reshaping of the abuttable area 101. The outwardly (from the flow channel) bulging areas 11a, 11b may provide for the axial movement of the annular element 12 by an enhanced flexibility of the hollow flexible body 19. Thus, the repositioning and/or reshaping of the abuttable area 101 may be conducted without causing strain or stress which may lead to lasting deformation or wear. For medical use, wear, such as cracks in the conduit could be an issue since it may increase the risk for contamination. Deformation of wear may also have affect the reliability of the equipment, e.g., could break down in critical situations. Such drawbacks are effectively avoided by the invented valve mechanism.

In this illustrated example, the valve 1 is closed by having an abuttable area 101 abutting an upstream area of the valve seat 13. An advantage of having the closing taking place at an upstream location of the valve seat 13 is that the force of the flow may help to close the valve 1, thus a faster closing response may be provided. Further, due to the force of the flow, the sealing capability of the valve in this example may be increased. A further advantage is the easy control of the valve and the forces are more handable.

Fig 1B illustrates a cross-section of a valve 1 from another angle. In this figure, an exemplary design of the struts 15, 16 for holding the valve seat 13 in its position in the flow channel is illustrated.

Fig 1C, show an illustration of a valve 1 from the outside. Here the annular element 12 and it position at the hollow flexible body 12 and the outwardly bulging areas, 11a, 11b area illustrated. A minimotor connected to conduct the movement of the annular element 12 is also illustrated.

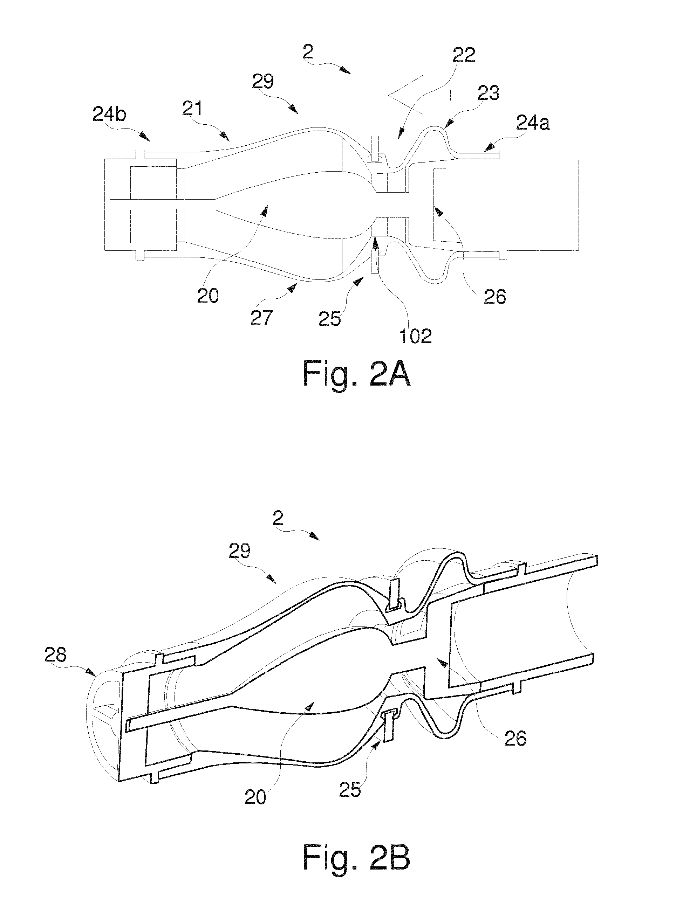

Fig2. 2A and 2B illustrate another example of a valve 2, The valve is constructed using a hollow flexible body 29 having a waist area 22. The hollow flexible body 29 is fastened at to fixed endpoints 24a, 24b. An axially movable annular element 25 is arranged within a portion of the waist area 22. Additionally and/or alternatively, in some examples, an outwardly bulging area 23 may be provided to increase the repositioning and/or reshaping of an abuttable area 102. The bulging area 23 has a curved inner surface.

The flow through the flow channel of the illustrated valve 2 is controlled by axially moving the annular element 25 in the flow direction. The valve 2 is closed by having the abuttable area 102 abutting an upstream area of a valve seat 20.

Portions of the wall 21 of the hollow flexible body 29 are gradually more rigid from a thinner area 27 and towards one of the fixed endpoints 24b. In one example, the gradual rigidity is obtained by gradually making the wall thicker. Additionally and/or alternatively in some examples, the wall 21 is also made more rigid from the thinner area 21 and towards the abuttable are 102.

The increased rigidity keep the walls from collapsing while still providing flexibility in a thinner area 27. Thus the repositioning and/or reshaping of the abuttable area 102 may be done without causing strain or stress in the material of the walls.

Additionally and/or alternatively, in some examples of the valve, a tubular element 26 may be provided that extend into the flow channel of the valve. By having an end surface of the tubular element 26 abutting a part of the bulging area 23 when the vale is in an open position flow into the bulging area 23 is avoided. Thus, less turbulence in the flow of a fluid through the flow channel of the valve may be obtained.

In some examples of the valve, the tubular element 26 may have a strut in one end to hold the valve seat 20. A further strut 28 may be provided downstream of the valve seat 20.

Additionally a shoulder may be provided at an upstream area of the valve seat 20. The shoulder is configured to accommodate the abuttable area 102 of the hollow flexible body 29 when abutting the valve seat 20. Thus increased sealability, when the valve is in a closed position, may be obtained. Hence this configuration may improve the sealability of the closed valve.

Some advantages with the design of the disclosed valve are an improved internal fluid-dynamic shape which provides a low pressure drop and more handable forces.

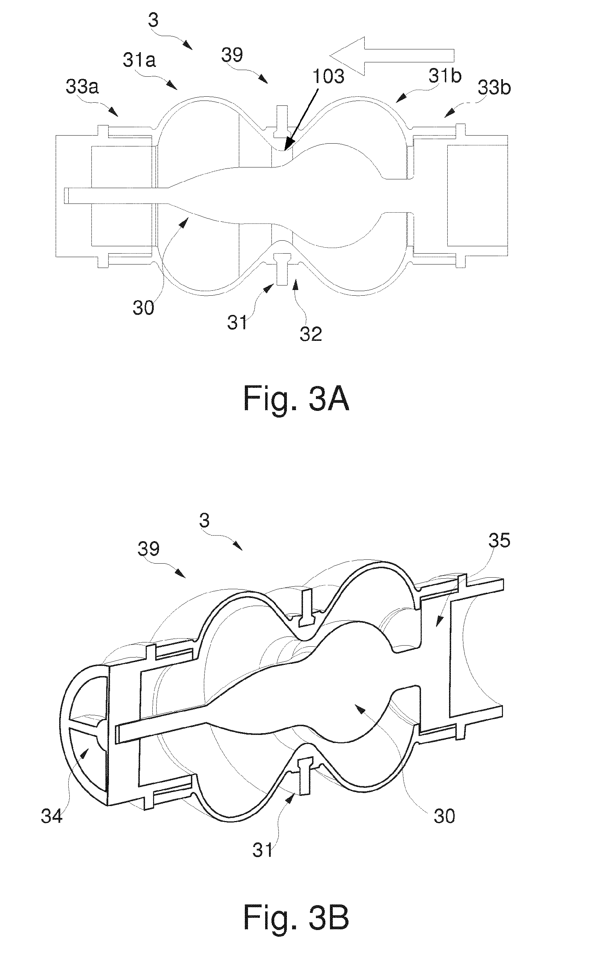

Figs. 3A and 3B illustrate an example of a valve 3. The valve mechanism is similar to the valve illustrated in Figs. 1A to 1C. The valve is constructed using a generally dumbbell shaped hollow flexible body 39 having a waist area 32 and two outwardly bulging areas 31a, 31b with a curved inner surface. The hollow flexible body 39 is fastened at to fixed endpoints 33a, 33b.

The bulging areas are provided to enhance the repositioning and/or reshaping of the abuttable area 103 when moving an annular element 31 in an axial direction of the flow through the flow channel.

In this example a centrally located valve seat 30 has a pear like shape and closing of the valve is made by moving the annular element 31 so that the repositioned and/or reshaped abuttable area 103 is abutting a downstream area of the valve seat 30.

This way of closing the valve may improve the response of the valve when opening since the force of the flow will move in the same direction as the annular element 31. The arrow indicates the flow of a fluid through a flow channel of the hollow flexible body 39.

The pear-shaped valve seat 30 may be hold in a position at the center of the flow channel by at least one strut 35 or 34.

Some advantages with this configuration of valve 3 are an improvement in the fluid-dynamic shape leading to a lower pressure drop and more handable force.

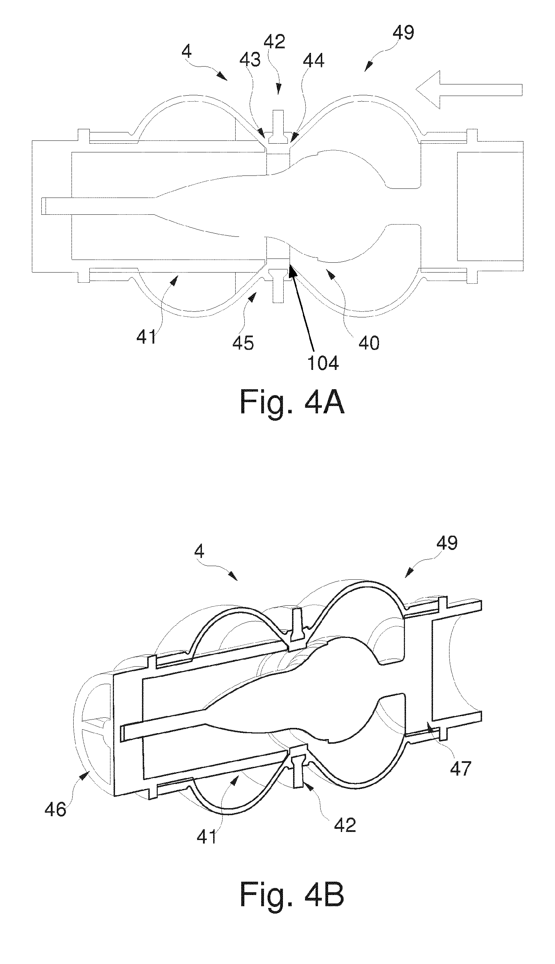

Figs. 4A and 4B illustrate a further example of a valve 4. The valve works similar to the example illustrated in Figs. 1A to 1C and Fig. 3A and 3B. In this example a tubular element 41, such as a symmetrical silicone tube, is extended into the flow channel of a hollow flexible body 49. In this example, the hollow flexible body 49 has two bulging areas connected by a waist area 42. By having the abuttable area 104 sealingly abutting an end surface 43 of the tubular element 41, when the valve 4 is in an open position, fluids are prevented from entering the space of the bulging area. Thus less turbulence in the flow may be provided since the bulging area is sealingly covered from a flow of a fluid.

A similar solution may be provided for a valve shown in Fig 1A to 1C, where a tubular element may extend into the flow channel of a hollow flexible body upstream as in Figs. 2A and 2B.

The valve seat 40 may be provided with a shoulder to accommodate an abuttable area 44 when repositioned and/or reshaped by an annular element 45 to a closed position. In the example shown, the annular element 45 is circumferentially positioned around a portion of the waist area 41.

The valve seat 40 may be held in a central position of the flow channel by at least one strut 46, 47.

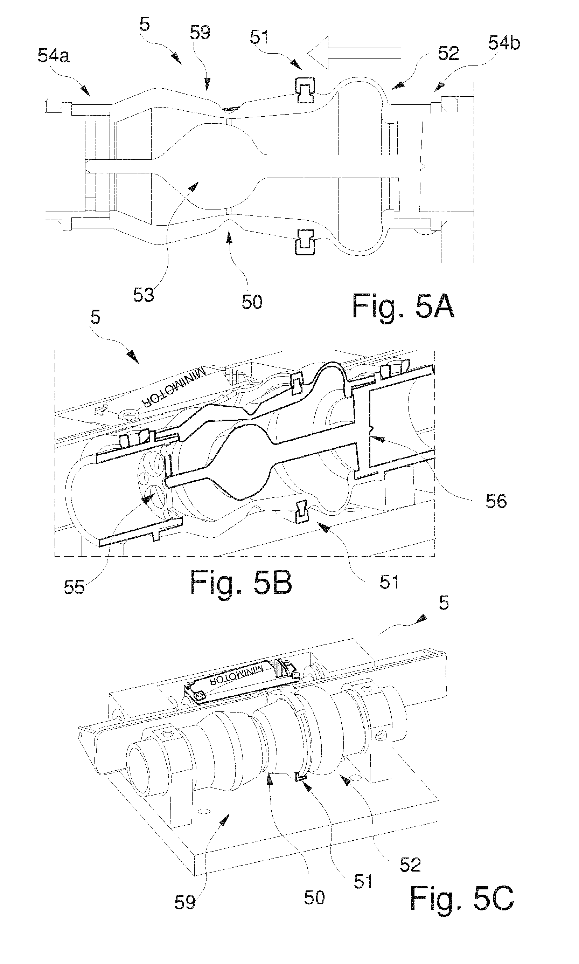

Figs. 5A to 5C illustrate a further examples of valve 5.

In one example, the abuttable area 50 of the hollow flexible body 59 is configured to controllably collapse against at valve seat 53 by an axial movement of an annular element 51. The annular element 51 is circumferential positioned between the abuttable area 50 and at least one of the fixed endpoints 54a, 54b, in one example, between the abuttable area 50 and the fixed endpoint 54b to have a smooth inner surface downstream a valve seat 53.

Additionally, in some examples of the valve 5 an outwardly bulging area 52 may be provided to enhance the axial movement of the annular element 51 without causing any stress or strain in the material of the hollow flexible body 59.

The valve seat 53 may have any shape previously described. The valve seat 53 may be hold in its centre position of the flow channel by at least one strut 55, 56.

One advantage with this example is the high fluid-dynamic shape providing low turbulence and low pressure drop.

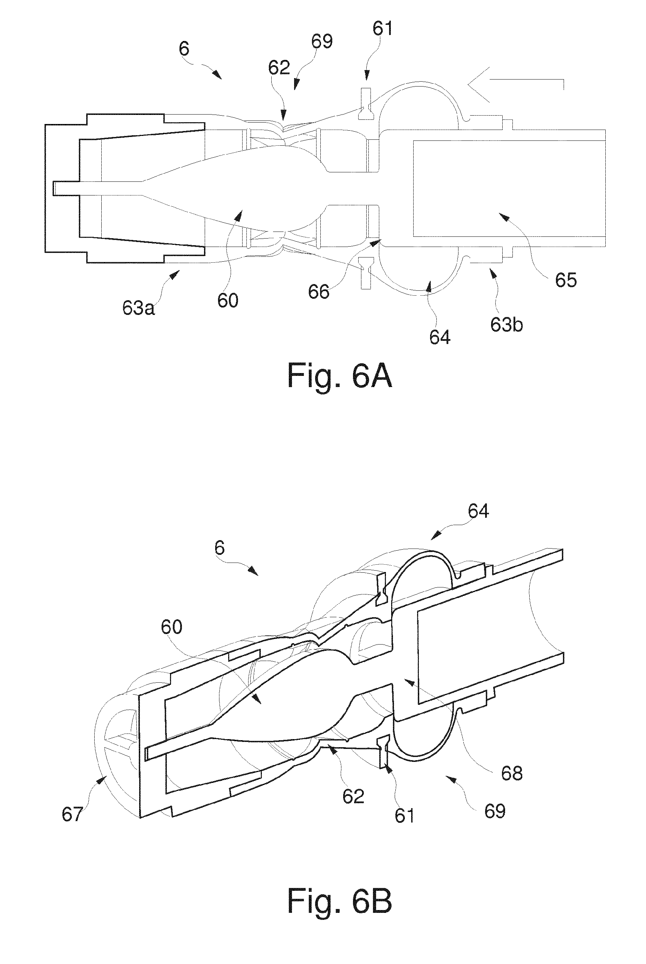

Figs. 6A and 6B illustrate an additional example of a valve 6 similar to the valve in fig. 5A and B. In this example a tubular element 65 is positioned into the flow channel of the hollow flexible body 69. The tubular element has similar advantages as the tubular element in Figs. 2A and 2B. Here, an end surface 66 of the tubular element 65 abuts an area of the inner surface of the hollow flexible body when the valve is in an open position. Thus fluid is prevented from entering the bulge area 64. The inner surface is held in an abutting positing by an annular element 61.

By moving the annular element axially in a direction of the flow the flow through the flow channel may be controlled by controllably collapsing the abuttable area 62 towards the valve seat 60.

The valve seat 60 is held in place by at least one strut 67, 68. And the hollow flexible body 69 is fastened at the fixed endpoints 63a, 63b.

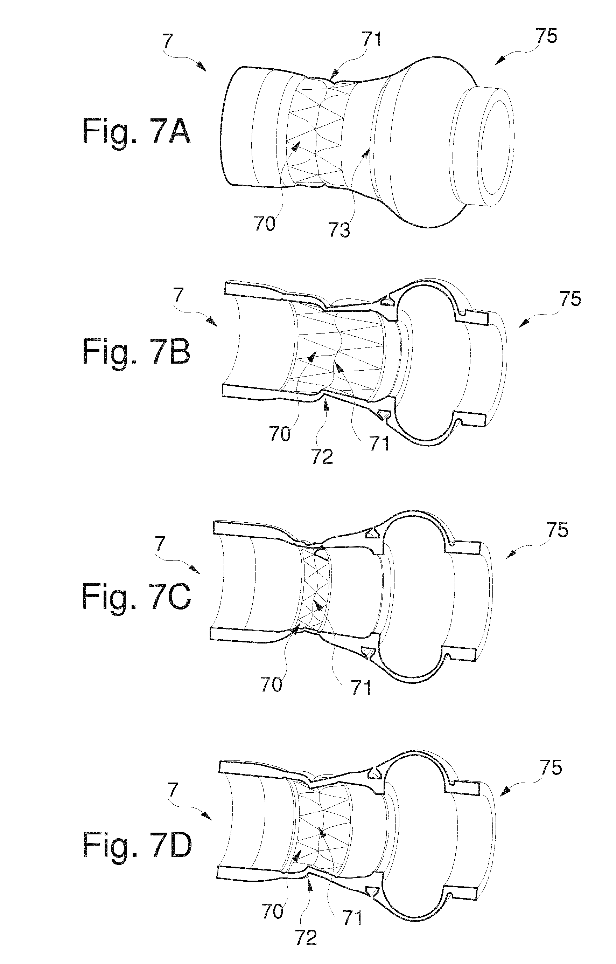

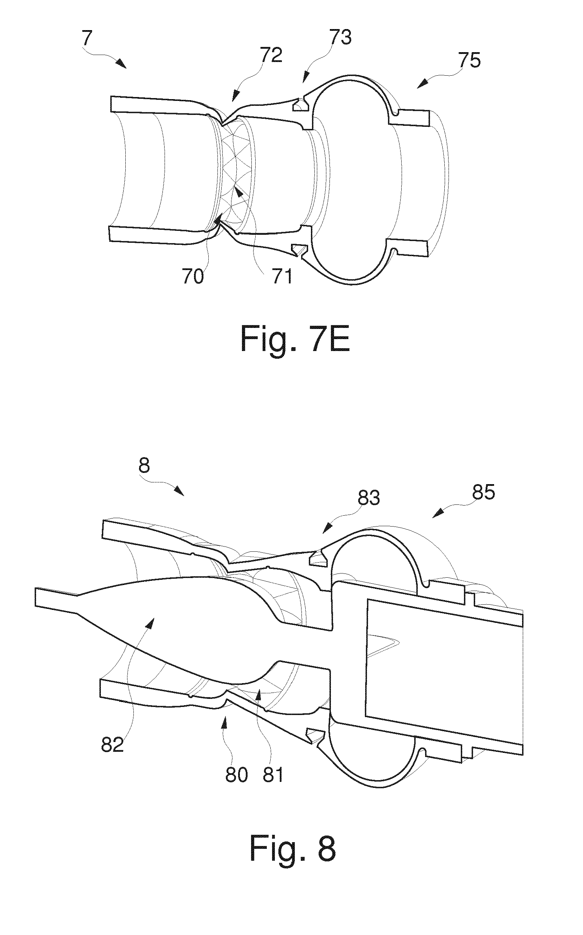

Figs. 7A to 7E illustrate a hollow flexible body 7 of a valve. The hollow flexible body 7 has an advantageous configuration of a collapsible and abuttable area 71. In one example, the abuttable area 71 has diamond shaped zones 70 along the circumference of the abuttable area 71 with a notch 72 in the middle. For illustrating purposes the position of an annular element 73 and a bulge area 75 is shown.

When the abuttable area is collapsed there may be no great stress or strain in the material of the hollow flexible body since the material will be twined along notch of the inner diameter, such as in a sinusoid. Thus the material itself needs to be minimally compressed to accommodate the material when collapsed to a smaller inner diameter. Apart from lowering the stress and strain of the material, this may further increase the sealability and the control of the collapse. The stress and strain may be lowered since there will be no compression of the material when collapsed according to this principle. Further, this may provide an improvement of the control of a flow through the valve.

Figs. 7A to 7E are showing different shapes, and angles of the diamond shapes that may be used for different kinds of configurations of a valve. The configuration used may depend on responses needed, sealability, the material and the dimensions of the valve and flow channel.

Fig 8. illustrates an example of a valve 8 which is similar to the valve in Figs. 6A and 6B. In this example the abuttable area 80 being collapsible has diamond shaped zones 81, according to the principle of the abuttable area previously described for Figs. 7A to 7E.

Alternatively the principle of the configuration of an abuttable area according to Figs. 7A to 7E may be used in conjunction with any valve described herein.

Further, the valve described herein may be constructed using materials of the hollow flexible body and the valve seats which are autoclavable. Alternatively, the construction material of the hollow flexible material and the valve seats may be disposable. Alternatively, the valve may comprise parts being autoclavable combined with parts being disposable. Examples of such autoclavable materials include silicone rubber, stainless steel etc.

An advantage according to one example is to improve positioning of a valve between two non-flexible in- and outlet channels. Additionally, bulging areas may be utilized so that the axial movement of an annular element repositioning and/or reshaping an abutting area may be carried out. Thus, strain in the material of the hollow flexible body and/or on the mountings of the non-flexible in- and outlet channels is avoided. Strain or stress could cause lasting deformation or wear. For medical use wear such as cracks in the conduit could be an issue since it may increase the risk for contamination. Deformation of wear may also affect the reliability of the equipment, e.g., the valve may malfunction in critical situations. Such drawbacks are effectively avoided by the herein described valve.

Further the valve provides for an improved pressure drop and less turbulence of the flow by providing an improved fluid-dynamic shape for the flow through the valve. Further the principles of the described valve provides for an increased controllability of the flow through the flow channel.

The principle of the function of the different parts of the described device may be regarded as steps for a method to mechanically control a flow in a flow channel, such as a flow channel of a medical ventilator.

As will be appreciated by one of skill in the art, the present invention may be embodied as device, system or method.

While several examples of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A valve for a medical ventilator comprising:
- a hollow flexible body having at least one outwardly bulging area with a curved inner surface; and an inwardly protruding abuttable area positioned between two fixed end points;
- a flow channel located inside said hollow flexible body for passage of a fluid between an inlet and an outlet;
- a valve seat centrally positioned in said flow channel;
- an annular element circumferentially arranged around said hollow flexible body;
wherein said annular element is arranged to be movable in an axial direction along said flow channel by an actuator unit, and whereby said abuttable area is at least partially repositioned and/or reshaped to control a flow of said fluid through said flow channel.

2. The valve according to claim 1, wherein said hollow flexible body having an outside waist area positioned between said two fixed end points.

3. The valve according to claim 1 or 2, wherein said bulging area has a substantially constant wall thickness at a central portion.

4. The valve according to any of claims 1 to 3, wherein said inner diameter of said flexible hollow body has en increased inner diameter at said bulging area.

5. The valve according to any of claims 1 to 4, wherein said hollow flexible body having walls gradually more rigid towards at least one of said two fixed end points.

6. The valve according to any of claims 1 to 5, wherein said hollow flexible body having walls gradually thicker towards at least one of said two fixed end points.

7. The valve according to any of claims 1 to 6, wherein said abuttable area is configured to, after reposition and/or reshaping, sealingly abut said valve seat.

8. The valve according to any of claims 1 to 7, wherein said annular element is circumferentially arranged outside a portion of said abuttable area.

9. The valve according to any of claims 1 to 8, wherein said abutting area is arranged to axially abut said valve seat from upstream or downstream upon repositioning and/or reshaping said abuttable area.

10. The valve according to any of claims 1 to 9, wherein said valve seat is pear shaped.

11. The valve according to any of claims 7 to 10, wherein said valve seat has a shoulder to accommodate said abutting area when closing said valve.

12. The valve according to any of claims 1 to 7, wherein said annular element is circumferentially arranged around said hollow flexible body between said abuttable area and one of said two fixed end points.

13. The valve according to any of claims 1 to 7 or 11, wherein said abuttable area, upon axial movement of said annular element, is configured to be in a radially collapsed state when sealingly abut said valve seat and/or to be in an uncollapsed state when said valve is open.

14. The valve according to claim 13 wherein said abuttable area has diamond shaped zones with a central notch.

15. The valve according to claim 14, wherein said notch of said diamond shaped zones is configured to provide a twining shape along the inner diameter of the abuttable area when in said collapsed state.

16. The valve according to any of claims 1 to 15, wherein a tubular element is extending into said flow channel downstream and/or upstream, and said tubular element is having an end surface arranged to abut an inner wall of said bulging area or to abut said abuttable area when said valve in an open state.

17. Use of an expiratory valve of claims 1 to 16 in a medical ventilator.
